# EUROPÄISCHE PATENTSCHRIFT

(11) **EP 0 789 029 B1**
(45) Veröffentlichungstag und Bekanntmachung des Hinweises auf die Patenterteilung: **10.09.2003**
(21) Anmeldenummer: 97101308.1
(22) Anmeldetag: 29.01.1997
(51) Int. Cl.: C07K 5/02, C07C 227/42, C07C 229/36

(54) **Verbessertes Verfahren zur Herstellung von reinem (S,S)-N-(1-Ethoxycarbonyl-3-phenylpropyl)-alanin)**
Improved procedure for the preparation of pure(S,S)-N-(1-Ethoxycarbonyl-3-phenylprophyl)-alanin).
Procédé amélioré de la préparation de (S,S)-N-(1-Ethylcarbonyl-3-phenylprophyl)-alanine)pur.

(30) Priorität: 05.02.1996 AT 19996
(43) Veröffentlichungstag der Anmeldung: 13.08.1997
(73) Patentinhaber: DSM Fine Chemicals Austria Nfg GmbH & Co KG, 4021 Linz (AT)
(72) Erfinder: Hackl, Kurt Alfred, Dr.Dipl.-Ing., 4020 Linz (AT); Schaller, Josef, 4020 Linz (AT)
(74) Vertreter: Lindinger, Ingrid

(56) Entgegenhaltungen:
- EP-A- 0 190 687
- EP-A- 0 215 335
- US-A- 4 542 234

## Beschreibung

(S,S)-N-(1-Ethoxycarbonyl-3-phenylpropyl)alanin (ECPA) ist eine wesentliche Vorstufe in der Synthese von verschiedenen blutdrucksenkenden Wirkstoffen vom Typ der Angiotensin Converting Enzyme-Inhibitors (ACE-Hemmer), wie etwa Enalapril, Enalaprilat, Ramipril, Delapril u. a.. In der Literatur sind bereits mehrere Herstellungsverfahren für ECPA beschrieben. ECPA wird beispielsweise durch Hydrierung von (S,S)-N-(1-Ethoxycarbonyl-3-oxo-3-phenylpropyl)-alaninester (EOPAE) erhalten. Dieser Teilschritt in der Synthese von ACE-Hemmern ist bereits aus der Literatur, beispielsweise aus Tetrahedron Letter, Vol. 25 (11) S. 1143 - 1146, 1984, bekannt.

Auch in EP 0 215 335, das die Herstellung von (S,S)-N-(1-Ethoxycarbonyl-3-phenylpropyl)-alanin-prolin beschreibt, wird auf diese Herstellvariante von ECPA, das eine Vorstufe dazu ist, hingewiesen. Desweiteren beschreibt EP 0 215 353 die Herstellmöglichkeit von ECPA durch asymmetrische Addition von Ethyl-β-benzoylacrylat und einem Alkalimetallsalz von Alanin.

Eine weitere Herstellungsmöglichkeit geht, gemäß DE-OS 35 42 735, von Ethyl-2-oxo-4-phenylbutyrat aus, das mit L-Alanin in Anwesenheit von Reduktionsmitteln, wie Natriumcyanoborhydrid oder Wasserstoff und einem Katalysator und einem Trocknungsmittel in einem polaren Lösungsmittel zu ECPA umgesetzt wird. Aus Organic Preparations and Procedures Int. 20 (2) 109- 115 (1988) ist eine dritte Variante bekannt, wonach ein Ethylester des Alanins entweder mit tert. Butyl-2-brompropionate oder Benzyl-2-brompropionat in Anwesenheit von Triethylamin mittels Rückflußkochen in CH₃CN umgesetzt wird. Der so erhaltene Ester wird sodann mittels HCI/p-Dioxan oder durch katalytische Hydrogenolyse mit Ethanol in Anwesenheit von 10 % Pd/C in ECPA überführt.

US 4,542,234 beschreibt ein Verfahren zur Abtrennung von (S,S)-Diastereomeren, wie etwa von ECPA, aus einer Mischung der (S,S)- und (R,R)-Diastereoisomeren. Diese Trennung erfolgt dadurch, dass die (S,S)-Verbindung in ein Salz, beispielsweise ein Maleat überführt wird, wodurch dieses ausfällt und durch Filtration isoliert werden kann. Abschließend erfolgt die Rückführung in die freie Säure. Das Problem bei diesen Verfahren liegt jedoch in der Isolierung des Produktes und zwar in Hinsicht auf die Reinheit des erhaltenen ECPA. Das nach Zusatz einer Base entweder ausfallende oder durch Extraktion erhaltene Rohprodukt enthält oft über 5 Gew.% an Verunreinigungen und muß daher noch durch Kristallisation, beispielsweise wie im Stand der Technik beschrieben, mit Ethylacetat gereinigt werden. Unter den bisher bekannten Reaktionsbedingungen, tritt jedoch das Hydrolyseprodukt (S,S)-N-(1-Carboxyl-3-phenylpropyl) alanin als Nebenprodukt auf, das auch durch wiederholte Umkristallisation aus Ethylacetat nicht beseitigt werden kann.

Aufgabe der vorliegenden Erfindung war es demnach, ein verbessertes Verfahren insbesondere zur Aufarbeitung der Reaktionslösungen zu finden, das die Bildung des Hydrolyseproduktes verhindert, bzw. die weitgehende Entfernung aus dem gewünschten Endprodukt ermöglicht.

Unerwarteterweise konnte diese Aufgabe ohne Verwendung eines organischen Extraktiosmittels, durch eine Umkristallisation aus Wasser gelöst werden.

Gegenstand der vorliegenden Erfindung ist demnach ein verbessertes Verfahren zur Herstellung von reinem (S,S)-N-(1-Ethoxycarbonyl-3-phenylpropyl)-alanin, das dadurch gekennzeichnet ist, dass verunreinigtes, rohes (S,S)-N-(1-Ethoxycarbonyl-3-phenylpropyl)-alanin, erhalten durch katalytische Hydrierung von (S,S)-N-(1-Ethoxycarbonyl-3-oxo--phenylpropyl)-alaninbenzylester in Essigsäure unter Zusatz katalytischer Mengen von Schwefelsäure, in Wasser suspendiert, durch Erhitzen auf Siedetemperatur gelöst und durch Abfiltrieren von unlöslichen Verunreinigungen abgetrennt und im Anschluss daran das gewünschte Endprodukt durch Abkühlen ausgefällt, abfiltriert und getrocknet wird.

Das erfindungsgemäße Verfahren geht von verunreinigtem, rohem (S,S)-N-(1-Ethoxycarbonyl-3-phenylpropyl)-alanin aus, das gemäß dem Stand der Technik durch katalytische Hydrierung von (S,S)-N-(1-Ethoxycarbonyl-3-oxo--phenylpropyl)alaninbenzylester in Essigsäure unter Zusatz katalytischer Mengen von Schwefelsäure erhalten wurde. Das verunreinigte ECPA wird dabei erfindungsgemäß in Wasser suspendiert und durch Erhitzen auf Siedetemperatur gelöst. Bevorzugt wird in so viel Wasser suspendiert, sodass sich das gesamte ECPA löst. Zu große Wassermengen sind nicht zu empfehlen, da dies zu mehr Verlust bei der Umkristallisation führt. Die heiße Lösung wird sodann filtriert, um unlösliche Verunreinigungen abzutrennen. Im Anschluß daran wird unter Rühren auf etwa 0 bis 20°C, bevorzugt 5 bis 15°C gekühlt, das ausgefallene reine ECPA abfiltriert oder abgesaugt und getrocknet.

Bevorzugt wird von einer beispielsweise gemäß Tetrahedron Letters, Vol. 25 (11), S. 1143 - 1146, 1984 oder gemäß Beispiel 12 aus EP- 0 190 687, durch katalytische Hydrierung von EOPAE in Essigsäure unter Zusatz katalytischer Mengen an Schwefelsäure erhaltenen Hydrierlösung ausgegangen. Zur Isolierung des gewünschten Endproduktes wird dabei zuerst der Katalysator abgetrennt und das Lösungsmittel Essigsäure destillativ, beispielsweise durch Abrotieren im Vakuum, entfernt. Der so erhaltene ölige Rückstand wird sodann in Wasser aufgenommen, wobei bevorzugt soviel Wasser zugegeben wird, dass eine 40 bis 70 %ige besonders bevorzugt eine 50 bis 60 %ige Lösung, bezogen auf das im Rückstand enthaltene Sulfat, erhalten wird. Anschließend wird durch den Zusatz einer Base ein pH-Wert von etwa 2 bis 5, bevorzugt von etwa 3 bis 4, eingestellt. Als Base eignen sich bevorzugt Natriumhydroxyd, Kaliumhydroxyd, Natrium- oder Kaliumcarbonat oder -hydrogencarbonat, wobei auch Mischungen derselben eingesetzt werden können. Die so erhaltene Lösung oder Suspension wird sodann unter Rühren auf etwa 0 bis 20°C, bevorzugt 5 bis 15°C, für eine Zeitraum von etwa 10 bis 120 Minuten gekühlt und das ausgefallene Produkt abgesaugt oder abfiltriert. ECPA wird dann wie oben beschrieben, in Wasser suspendiert und durch Erhitzen auf Siedetemperatur gelöst. Die Menge an Wasser kann dabei variieren und hängt von der zu lösenden Menge an ECPA ab. Es sollte dabei wiederum bevorzugt soviel Wasser verwendet werden, dass sich das gesamte ECPA gerade löst. Die heiße Lösung wird sodann analog oben beschriebenem Verfahren zur Abtrennung von unlöslichen Verunreinigungen filtriert, etwa über eine Glassinternutsche, und im Anschluß daran unter Rühren auf etwa 0 bis 20°C, bevorzugt 5 bis 15°C gekühlt. Das ausgefallene Produkt ECPA wird abfiltriert oder abgesaugt und bevorzugt im Vakuumtrockenschrank bei 30 bis 50°C, bevorzugt bei etwa 40°C, getrocknet.

Durch das erfindungsgemäße Verfahren wird ECPA in einer Ausbeute von über 85% mit einer Reinheit von bis zu 99,9 % erhalten, wobei der Anteil an dem Hydrolyseprodukt (S,S)-N-(1-Carboxyl-3-phenylpropyl)alanin sowie an dem R,S-Isomeren jeweils unter 0,1 % liegt.

### Beispiel 1

80 g (0,21 mol) (S,S)-N-(1-Ethoxycarbonyl-3-oxo-3-phenylpropyl)alaninbenzyl-ester wurden in 900 ml Essigsäure gelöst und mit 21,6 g (0,21 mol) konzentrierter Schwefelsäure versetzt.

Anschließend wurden 5 Stück Palladiumkatalysatoren der Fa. Degussa (Festbett groß) zugegeben und bei 35 °C und 8 L Umlauf/min 2 Stunden lang hydriert, bis keine H₂-Aufnahme mehr erfolgte.

Von der so erhaltenen Hydrierlösung wurde Essigsäure im Vakuum (etwa 10 mbar) bei 40°C abrotiert, worauf 121,3 g Rohprodukt erhalten wurden, die sodann mit 200 ml Wasser verdünnt wurden. Anschließend wurden 500 ml 1N NaOH zugegeben, worauf sich ein pH-Wert von 3,5 einstellte. Die Reaktionsmischung wurde dann 1 Stunde unter Eiskühlung auf 10°C unter Rühren gekühlt und das ausgefallene Produkt abgesaugt. Der Feststoff wurde in 800 ml Wasser heiß gelöst, heiß über eine Glassinternutsche filtriert und anschließend 1 Stunde unter Eiskühlung auf 10°C unter Rühren gekühlt. Das ausgefallene (S,S)-N-(Ethoxycarbonyl-3-oxophenylpropyl)alanin (81,7 g) wurde abgesaugt und im Vakuumtrockenschrank bei 40°C getrocknet.

| | |
|---|---|
| Ausbeute | 51,1 g (87,7 % d. Th.) |
| Schmelzpunkt | 148,4 - 148,9°C |
| opt. Drehung | 24,24° (c = 1; MeOH) |
| HPLC-Gehalt | 99,88 FI% |
| Hydrolyseprodukt | < 0,1 FI% |

## Patentansprüche

1. Verfahren zur Herstellung von reinem (S,S)-N-(1-Ethoxycarbonyl-3-phenylpropyl)-alanin **dadurch gekennzeichnet, dass** verunreinigtes (S,S)-N-(1-Ethoxycarbonyl-3-phenylpropyl)-alanin, erhalten durch katalytische Hydrierung von (S,S)-N-(1-Ethoxycarbonyl-3-oxo--phenylpropyl)-alaninbenzylester in Essigsäure unter Zusatz katalytischer Mengen von Schwefelsäure, in Wasser suspendiert, durch Erhitzen auf Siedetemperatur gelöst und durch Abfiltrieren von unlöslichen Verunreinigungen abgetrennt und im Anschluß daran das gewünschte Endprodukt durch Abkühlen ausgefällt, abfiltriert und getrocknet wird.

2. Verfahren nach Anspruch 1, **dadurch gekennzeichnet, dass** das verunreinigte (S,S)-N-(1-Ethoxycarbonyl-3-phenylpropyl)-alanin in soviel Wasser suspendiert wird, dass sich das gesamte (S,S)-N-(1-Ethoxycarbonyl-3-phenylpropyl)-alanin beim Erhitzen auf Siedetemperatur gerade noch löst.

3. Verfahren nach Anspruch 1, **dadurch gekennzeichnet, dass** im Anschluß an eine katalytische Hydrierung von (S,S)-N-(1-Ethoxycarbonyl-3-oxo-3-phenylpropyl)alaninbenzylester in Essigsäure und unter Zusatz einer katalytischen Menge an Schwefelsäure nach beendeter Reaktion das Lösungsmittel Essigsäure destillativ entfernt, der ölige Rückstand in Wasser aufgenommen und durch Zusatz einer Base auf einen pH-Wert von 2 bis 5 eingestellt wird, worauf der dabei erhaltene Niederschlag an (S,S)-N-(1-Ethoxycarbonyl-3-phenylpropyl)alanin in Wasser suspendiert, durch Erhitzen auf Siedetemperatur gelöst, durch Abfiltrieren von unlöslichen Verunreinigungen abgetrennt wird und im Anschluß daran das gewünschte Endprodukt durch Abkühlen ausgefällt, abfiltriert und getrocknet wird.

4. Verfahren nach Anspruch 3, **dadurch gekennzeichnet, dass** der ölige Rückstand in soviel Wasser aufgenommen wird, dass eine 40 bis 70 %ige Lösung, bezogen auf das im Rückstand enthaltene Sulfat, erhalten wird.

5. Verfahren nach Anspruch 3, **dadurch gekennzeichnet, dass** als Base Natriumhydroxyd, Kaliumhydroxyd, Natrium- oder Kaliumcarbonat oder -hydrogencarbonat verwendet wird.

6. Verfahren nach Anspruch 3, **dadurch gekennzeichnet, dass** ein pH-Wert von 3 bis 4 eingestellt wird.

7. Verfahren nach Anspruch 3, **dadurch gekennzeichnet, dass** der nach Einstellung eines pH-Wertes von 2 bis 5 erhaltene Niederschlag an (S,S)-N-(1-Ethoxycarbonyl-3-phenylpropyl)-alanin in gerade soviel Wasser suspendiert wird, dass sich das gesamte (S,S)-N-(1-Ethoxycarbonyl-3-phenylpropyl)-alanin beim Erhitzen auf Siedetemperatur gerade noch löst.

## Claims

1. Process for the preparation of pure (S,S)-N-(l-ethoxycarbonyl-3-phenylpropyl)alanine, **characterized in that** contaminated (S,S)-N-(1-ethoxycarbonyl-3-phenylpropyl)alanine, obtained by catalytic hydrogenation of (S,S)-N-(1-ethoxycarbonyl-3-oxophenylpropyl)alanine benzyl ester in acetic acid with the addition of catalytic amounts of sulphuric acid, is suspended in water, dissolved by heating to the boiling temperature and separated off from insoluble impurities by filtration and subsequently the desired end product is precipitated out by cooling, filtered off and dried.

2. Process according to Claim 1, **characterized in that** the contaminated (S,S)-N-(1-ethoxycarbonyl-3-phenylpropyl)alanine is suspended in enough water so that all the (S,S)-N-(1-ethoxycarbonyl-3-phenylpropyl)alanine just still dissolves upon heating to the boiling temperature.

3. Process according to Claim 1, **characterized in that** after a catalytic hydrogenation of (S,S)-N-(1-ethoxycarbonyl-3-oxo-phenylpropyl)alanine benzyl ester in acetic acid and with the addition of a catalytic amount of sulphuric acid, when the reaction is completed the solvent acetic acid is removed by distillation, the oily residue is taken up in water and adjusted to a pH of from 2 to 5 by adding a base, and then the resulting precipitate of (S,S)-N-(1-ethoxycarbonyl-3-phenylpropyl)alanine is suspended in water, dissolved by heating to the boiling temperature, separated off from insoluble impurities by filtration, and then the desired end product is precipitated out by cooling, filtered off and dried.

4. Process according to Claim 3, **characterized in that** the oily residue is taken up in enough water so that a 40 to 70% strength solution, based on the sulphate present in the residue, is obtained.

5. Process according to Claim 3, **characterized in that** the base used is sodium hydroxide, potassium hydroxide, sodium carbonate or potassium carbonate or sodium hydrogencarbonate or potassium hydrogencarbonate.

6. Process according to Claim 3, **characterized in that** a pH of from 3 to 4 is set.

7. Process according to Claim 3, **characterized in that** the precipitate of (S,S)-N-(1-ethoxycarbonyl-3-phenylpropyl)alanine obtained after setting a pH of from 2 to 5 is suspended in just enough water for all of the (S,S)-N-(1-ethoxycarbonyl-3-phenylpropyl)alanine to just still dissolve upon heating to the boiling temperature.

## Revendications

1. Procédé pour la préparation de (S,S)-N-(1-éthoxycarbonyl-3-phénylpropyl)alanine pure, **caractérisé en ce que** la (S,S)-N-(1-éthoxycarbonyl-3-phénylpropyl)alanine souillée, obtenue par hydrogénation catalytique de l'ester benzylique de la (S,S)-N-(1-éthoxycarbonyl-3-oxophénylpropyl)alanine dans l'acide acétique avec addition de quantités catalytiques d'acide sulfurique, est mise en suspension dans de l'eau, dissoute par chauffage jusqu'à la température d'ébullition, séparée par filtration des impuretés insolubles et ensuite, le produit final souhaité précipite par refroidissement, est filtré et séché.

2. Procédé selon la revendication 1, **caractérisé en ce que** la (S,S)-N-(1-éthoxycarbonyl-3-phénylpropyl)alanine souillée est mise en suspension dans assez d'eau pour que toute la (S,S)-N-(1-éthoxycarbonyl-3-phénylpropyl)alanine soit juste dissoute par chauffage à la température d'ébullition.

3. Procédé selon la revendication 1, **caractérisé en ce que** suite à une hydrogénation catalytique de l'ester benzylique de la (S,S)-N-(1-éthoxycarbonyl-3-oxophénylpropyl)alanine dans l'acide acétique avec addition de quantités catalytiques d'acide sulfurique, à la fin de la réaction, on élimine le solvant, l'acide acétique, par distillation, on reprend le résidu huileux dans l'eau et on ajuste à un pH allant de 2 à 5 par addition d'une base, où le résidu obtenu de la (S,S)-N-(1-éthoxycarbonyl-3-phénylpropyl)alanine est mis en suspension dans l'eau, dissous par chauffage jusqu'à la température d'ébullition, séparé des impuretés insolubles par filtration et ensuite, le produit final précipite par refroidissement, est filtré et séché.

4. Procédé selon la revendication 3, **caractérisé en ce que** le résidu huileux est repris dans assez d'eau pour obtenir une solution de 40 à 70%, sur base du sulfate présent dans le résidu.

5. Procédé selon la revendication 3, **caractérisé en ce que** l'on utilise comme base, l'hydroxyde de sodium, l'hydroxyde de potassium, le carbonate ou l'hydrogénocarbonate de sodium ou de potassium.

6. Procédé selon la revendication 3, **caractérisé en ce que** l'on ajuste un pH allant de 3 à 4.

7. Procédé selon la revendication 3, **caractérisé en ce que** le résidu obtenu après l'ajustement du pH de 2 à 5, de la (S,S)-N-(1-éthoxycarbonyl-3-phénylpropyl)alanine, est mis en suspension dans juste assez d'eau pour que toute la (S,S)-N-(1-éthoxycarbonyl-3-phénylpropyl)alanine soit juste dissoute par chauffage jusqu'à la température d'ébullition.
